# EUROPEAN PATENT SPECIFICATION

(11) **EP 4 201 382 B1**
(45) Date of publication and mention of the grant of the patent: **08.10.2025**
(21) Application number: 22215686.1
(22) Date of filing: 21.12.2022
(51) Int. Cl.: A61F 9/008, A61F 9/009

(54) **OPHTHALMOLOGICAL DEVICE FOR PROCESSING A CURVED TREATMENT FACE**
OPHTHALMOLOGISCHE VORRICHTUNG ZUR BEARBEITUNG EINER GEKRÜMMTEN BEHANDLUNGSFLÄCHE
DISPOSITIF OPHTALMOLOGIQUE DE TRAITEMENT D'UNE FACE DE TRAITEMENT COURBE

(30) Priority: 23.12.2021 CH 0707932021
(43) Date of publication of application: 28.06.2023
(73) Proprietor: Ziemer Ophthalmic Systems AG, 2562 Port (CH)
(72) Inventor: RATHJEN, Christian, 28197 Bremen (DE); STEINLECHNER, Michael, 8006 Zürich (CH)
(74) Representative: Rentsch Partner AG

(56) References cited:
- US-A1- 2011 028 948
- US-A1- 2016 317 349
- US-A1- 2017 087 022
- US-A1- 2021 259 882

## Description

### Field of Disclosure

The present disclosure relates to an ophthalmological device for processing a curved treatment face in eye tissue. In particular, the present disclosure relates to an ophthalmological device comprising a laser source configured to generate a pulsed laser beam, a focussing optical module configured to make the pulsed laser beam converge onto a focal spot in or on the eye tissue, and a scanner system configured to move the focal spot to target locations in or on the eye tissue.

### Background of the Disclosure

For the purposes of working on eye tissue by means of a laser beam, a work region is scanned by laser pulses by virtue of the pulsed laser beam being deflected in one or more scan directions by means of suitable scanner systems and converged onto a focal spot by a focussing optical module. In general, movable mirrors are used to deflect the light beams and/or the laser pulses, for example femtosecond laser pulses, said movable mirrors being pivotable about one or two scan axes, for example by way of galvano scanners, piezo scanners, polygon scanners, or resonance scanners. Further scan components, such as divergence modulators or z-modulators, are known for positioning and moving the focal spot in the eye tissue with respect to further scan axes.

US 7,621,637 describes an apparatus for working on eye tissue, said apparatus having a base station with a laser source for producing laser pulses and a scanner, arranged in the base station, with movable deflection mirrors for deflecting the laser pulses in a scan direction. The deflected laser pulses are transferred via an optical relay system from the base station to an application head, the latter passing over a work region according to a scan pattern by means of a mechanically moved projection optical unit. According to US 7,621,637, in the application head, the deflection in the scan direction, which is much faster in comparison with the mechanical movement, is overlaid onto the mechanical movement of the projection optical unit and consequently onto the scan pattern thereof. A fast scanner system in the base station facilitates a fine movement of the laser pulses (micro-scan), which is overlaid on the scan pattern of the (mechanically) movable projection optical unit that covers a large work region, for example the entire eye.

US 2011/0028948 A1 describes an optical system for an ophthalmic surgical laser. According to US 2011/0028948 A1, the ophthalmic surgical laser system includes a laser source for producing a pulsed laser beam, an XY scanner for scanning the pulsed laser beam in XY directions transverse to a Z axis, a Z scanner for scanning the XY scanned laser beam along the Z axis, and an objective for focusing the XYZ scanned laser beam into a target region. US 2011/0028948 A1 further describes an embodiment with two Z scanners of different scanning ranges. The first Z scanner receives the laser beam from the laser source and scans a focal point along an optical axis of the laser delivery system within a Z scanning range of 0.05-1mm (or 1-5mm), e.g. for corneal surgery, with a first scanning time. The beam output by the first Z scanner is received by the XZ scanner. The beam output by the XY scanner is received by the second Z scanner which scans the focal point along the optical axis within a Z scanning range of 1-5mm (or 5-10mm), e.g. for an anterior segment surgical procedure, with a comparatively slower second scanning time.

For processing curved treatment faces in the eye tissue, e.g. for generating curved incision faces in the eye tissue, ophthalmological devices use scanner systems with multiple scan axes and various actuators for producing rotational and translational actuation associated respectively with the scan axes. With the increase of flexibility and performance, these scanner systems made it possible to define and produce three-dimensionally curved treatment faces and volumes of almost any possible shape in the eye tissue. Nevertheless, as the scanner systems and their actuators do have performance limits, there remains a risk that the processing of curved treatment faces in the eye tissue exceeds the performance limits of individual components of the scanner systems, thereby overcharging the scanner system, particularly by exceeding the capabilities of known z-scanners which modulate the focal depth in projection direction, which leads to undesired alteration of the intended treatment and possibly dangerous and harmful consequences to a patient's eye.

### Summary of the Disclosure

It is an object of the present disclosure to propose an ophthalmological device for processing a curved treatment face in eye tissue using a scanner system to move a focal spot of a pulsed laser beam, which device does not have at least some of the disadvantages of the prior art. Particularly, it is an object of the present disclosure to propose an ophthalmological device for processing a curved treatment face in eye tissue, which device avoids overcharging z-scanners which modulate focal depth.

According to the present disclosure, these objects are achieved by the features of the independent claims. Moreover, further advantageous embodiments emerge from the dependent claims and the description.

An ophthalmological device for processing a curved treatment face in eye tissue comprises a laser source configured to generate a pulsed laser beam; a focussing optical module configured to make the pulsed laser beam converge onto a focal spot in or on the eye tissue; a scanner system with a z-scanner, configured to modulate a depth of the focal spot along the projection axis, and an x/y-scanner system, configured to move the focal spot in directions normal to the projection axis; and a circuit configured to control the scanner system to move the focal spot to target locations on the curved treatment face along a processing path defined by treatment control data.

According to the present disclosure, the above-mentioned objects are particularly achieved in that the scanner system comprises a first z-scanner, configured to modulate a depth of the focal spot along the projection axis with first scan performance characteristics, indicating the dynamic depth scanning capabilities of the first z-scanner, a second z-scanner, configured to modulate the depth of the focal spot along the projection axis with second scan performance characteristics, indicating the dynamic depth scanning capabilities of the second z-scanner, whereby the dynamic depth scanning capabilities of the second z-scanner are greater than the dynamic depth scanning capabilities of the first z-scanner; and the circuit is further configured to determine from the treatment control data a depth scanning requirement, representing modulation of the depth of the focal spot along the processing path defined by the treatment control data, to divide the depth scanning requirement into a first depth scanning component for the first z-scanner and a second depth scanning component for the second z-scanner, to control the first z-scanner using the first depth scanning component, and to control the second z-scanner using the second depth scanning component.

In an embodiment, the circuit is configured to determine the first depth scanning component and the second depth scanning component, using the first scan performance characteristics and the second scan performance characteristics.

In an embodiment, the circuit is configured to determine from the treatment control data a spherical component of the curved treatment face and a complementary component for the curved treatment face, the complementary component complementing the spherical component to make up the curved treatment face, and to divide the depth scanning requirement into the first depth scanning component, as required for the modulation of the depth of the focal spot for the spherical component, and the second depth scanning component, as required for the modulation of the depth of the focal spot for the complementary component.

In an embodiment, determining the depth scanning requirement includes determining the required dynamics of the modulation of the depth of the focal spot along the processing path defined by the treatment control data; and the circuit is configured to determine the first depth scanning component using the first scan performance characteristics and the required dynamics, and to determine the second depth scanning component using the second scan performance characteristics and the required dynamics.

In an embodiment, the required dynamics comprise a required depth scanning speed, a required depth scanning frequency, a required amplitude of depth modulation at a particular speed of the depth modulation, a required amplitude of depth modulation at a particular frequency of the depth modulation, a required acceleration of the depth modulation, and/or a required speed of the acceleration of the depth modulation

In an embodiment, the circuit is configured to determine the depth scanning feasibility by checking whether the depth scanning requirement is achievable for the required dynamics, without exceeding the dynamic depth scanning capabilities of the first z-scanner and/ or the dynamic depth scanning capabilities of the second z-scanner, and to adjust the treatment control data to reduce or vary a speed of moving the focal spot along the processing path, in case the depth scanning feasibility is not affirmed.

In an embodiment, the circuit is configured to determine the depth scanning feasibility by computing a simulation of moving the focal spot along the processing path, defined by the treatment control data, using the first depth scanning component and the second depth scanning component.

In an embodiment, the circuit is configured to perform the depth scanning feasibility by controlling the scanner system to move the focal spot along the processing path, defined by the treatment control data, using the first depth scanning component and the second depth scanning component, while setting the laser source to a deactivated state and/or a reduced energy without any effect to the eye tissue.

In an embodiment, the first scan performance characteristics include a first maximum depth scanning speed or frequency; the second scan performance characteristics include a second maximum depth scanning speed or frequency which is faster than the first maximum depth scanning speed or frequency; and the circuit is configured to determine the first depth scanning component using the first maximum depth scanning speed or frequency, and to determine the second depth scanning component using the a second maximum depth scanning speed or frequency.

In an embodiment, the first scan performance characteristics include a first maximum amplitude of depth modulation at a particular speed or frequency of the depth modulation; the second scan performance characteristics include a second maximum amplitude of depth modulation at the particular speed or frequency of the depth modulation, the second maximum amplitude of depth modulation being smaller than the first maximum amplitude of depth modulation in a comparatively lower dynamic performance range, and the second maximum amplitude of depth modulation being greater than the first maximum amplitude of depth modulation in a comparatively higher dynamic performance range; and the circuit is configured to determine the first depth scanning component using the first maximum amplitude of depth modulation, and to determine the second depth scanning component using the second maximum amplitude of depth modulation.

In an embodiment, the first scan performance characteristics include a first maximum acceleration of the depth modulation, and/ or a first maximum speed of the acceleration of the depth modulation; the second scan performance characteristics include a second maximum acceleration of the depth modulation, greater than the first maximum acceleration of the depth modulation, and/or a second maximum speed of the acceleration of the depth modulation, greater than the first maximum speed of the acceleration of the depth modulation; and the circuit is configured to determine the first depth scanning component, using the first maximum acceleration or the first maximum speed of the acceleration, and to determine the second depth scanning component, using the second maximum acceleration or the second maximum speed of the acceleration.

In an embodiment, the ophthalmological device further comprises a patient interface having a central axis and being configured to fix the focussing optical module on the eye; and the circuit is further configured, in case of a tilt of the eye with respect to the central axis of the patient interface, or vice versa, to adapt the treatment control data to tilt the curved treatment surface corresponding to the tilt of the eye, prior to determining the depth scanning requirement, and to use the adapted treatment control data to determine the depth scanning requirement and divide the depth scanning requirement into the first depth scanning component and the second depth scanning component.

In an embodiment, the scanner system is configured to move the focal spot along a spiral-shaped processing path.

In an embodiment, the x/y-scanner system comprises a first x/y-scanner, configured to move the focal spot with a feed speed along a feed line of the processing path, and the x/y-scanner system comprises a second x/y-scanner, configured to move the focal spot with a scan speed, which is higher than the feed speed, along a scan line extending transversely with respect to the feed line of the processing path.

In an embodiment, the first z-scanner comprises a first actuator, the second z-scanner comprises a second actuator, and the circuit is configured to determine a phase difference between actuation by the first actuator and actuation by the second actuator, and to generate, for the first depth scanning component, a first control signal for the first actuator and, for the second depth scanning component, a second control signal for the second actuator, using the phase difference.

In addition to the ophthalmological device for processing a curved treatment face in eye tissue, the present disclosure further relates to a computer program product, particularly, a computer program product comprising a non-transitory computer-readable medium having stored thereon computer program code for controlling a processor of an ophthalmological device for processing a curved treatment face in eye tissue. The ophthalmological device comprises a laser source configured to generate a pulsed laser beam, a focussing optical module configured to make the pulsed laser beam converge onto a focal spot in the eye tissue, and a scanner system comprising a first z-scanner, configured to modulate a depth of the focal spot along the projection axis with first scan performance characteristics, indicating dynamic depth scanning capabilities of the first z-scanner, a second z-scanner, configured to modulate the depth of the focal spot along the projection axis with second scan performance characteristics, indicating dynamic depth scanning capabilities of the second z-scanner, whereby the dynamic depth scanning capabilities of the second z-scanner are greater than the dynamic depth scanning capabilities of the first z-scanner, and an x/y-scanner system configured to move the focal spot in directions normal to the projection axis. The computer program code is configured to control the processor such that the processor: uses treatment control data to control the scanner system to move the focal spot in the eye tissue to target locations along a processing path, defined by the treatment control data to process a curved treatment face in the eye tissue; determines from the treatment control data a depth scanning requirement, representing modulation of the depth of the focal spot along the processing path defined by the treatment control data; divides the depth scanning requirement into a first depth scanning component for the first z-scanner and a second depth scanning component for the second z-scanner; controls the first z-scanner using the first depth scanning component; and controls the second z-scanner using the second depth scanning component.

### Brief Description of the Drawings

The present disclosure will be explained in more detail, by way of example, with reference to the drawings in which:
- Figure 1:: shows a block diagram that schematically illustrates an ophthalmological device for processing a curved treatment face in eye tissue with a pulsed laser beam, said device comprising a focusing optical module for focusing the pulsed laser beam, and a scanner system for moving the focal spot to target locations in or on the eye tissue.
- Figure 2:: shows a schematic top view of a processing path with a series of partially overlapping focal spots moved by the scanner system along the processing path.
- Figure 3:: shows a schematic cross-sectional view of superposed processing paths with focal spots moved by the scanner system along the processing paths, whereby the focal spots partial overlap along the processing paths and across the superposed processing paths.
- Figure 4:: shows a schematic three-dimensional view of volume treatment with a composite processing path having focal spots partially overlapping along scan lines, across neighbouring scan lines moved along a feed line, and across superposed scan lines moved along the feed line.
- Figure 5:: shows a schematic top view of a spiral shaped processing path for processing a curved treatment face in eye tissue.
- Figure 6:: shows a schematic three-dimensional view of a section of a spiral shaped processing path for processing a curved treatment face in eye tissue.
- Figure 7:: shows a schematic three-dimensional view of a section of composite processing path, with a spiral shaped feed line and an overlaid scan line, for processing a curved treatment face in eye tissue.
- Figure 8:: shows a schematic three-dimensional view of a curved treatment face in the eye tissue, whereby the eye and a patient interface are aligned.
- Figure 9:: shows a schematic three-dimensional view of a curved treatment face in the eye tissue, whereby the eye is tilted with respect to the patient interface (or vice versa).
- Figure 10:: shows a graph illustrating the depth scanning requirements of moving the focal spot along a processing path for processing a curved treatment face, depicting the required modulation of depth of the focal spot along the projection axis depending on the scanning frequency, whereby a frequency threshold divides the depth scanning requirements into a first depth scanning component and a second depth scanning component.
- Figure 11:: shows a graph illustrating a comparison of the depth scanning requirements of moving the focal spot along a processing path for processing a curved treatment face, and the dynamic depth scanning capabilities of a first z-scanner and of a second z-scanner, depicted as maximum amplitude of depth modulation for a particular scanning frequency.
- Figure 12:: shows a flow diagram illustrating an exemplary sequence of steps for processing a curved treatment face in eye tissue, whereby the depth scanning requirement is dived into a first depth scanning component for a first z-scanner and a second depth scanning component for a second z-scanner.

### Detailed Description of the Embodiments

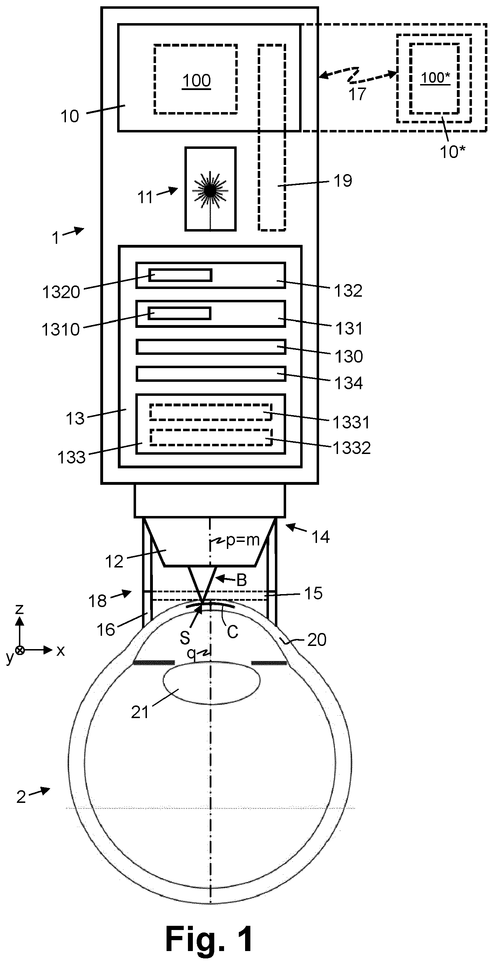

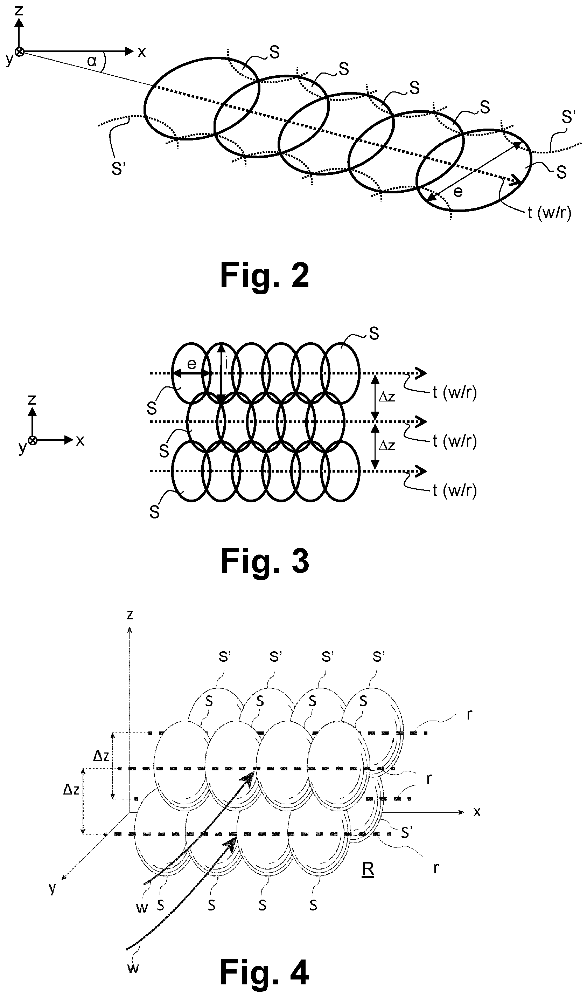

In Figure 1, reference numeral 1 relates to an ophthalmological device for processing a curved treatment face C in eye tissue using a pulsed laser beam B. The eye tissue comprises the cornea 20, the lens 21, or other tissue of the eye 2. Specifically, the ophthalmological device 1 is configured to process the curved treatment face C for irradiating the eye issue or for cutting the eye tissue, whereby in the latter case the curved treatment face C constitutes a curved incision face C. The person skilled in the art will understand that by generating two or more curved incision faces C, it is possible to cut three-dimensional tissue pieces, e.g. lenticular tissue pieces, which can be removed from the eye, e.g. a lenticular tissue piece of the cornea 20 which is removed from within the cornea 20 for refractive correction of the cornea 20. The person skilled in the art will further understand that by (partially) overlaying several curved treatment faces C or curved incision faces C, respectively, it is possible to achieve three-dimensional volume treatment of the eye tissue, e.g. for generating a void volume R inside the cornea 20, as illustrated schematically in Figure 4, for refractive correction of the cornea 20 and/or for other purposes, such as for inserting implants into the cornea 20.

As illustrated schematically in Figure 1, the ophthalmological device 1 comprises a laser source 11 for generating the pulsed laser beam B, a focusing optical module 12 for focusing the pulsed laser beam B onto a focal spot S in or on the eye tissue, and a scanner system 13 for moving the focal spot S to target locations in and/or on the eye tissue.

The ophthalmological device 1 further comprises an electronic circuit 10 for controlling the laser source 11 and the scanner system 13. The electronic circuit 10 implements a programmable control device and comprises e.g. one or more processors 100 with program and data memory and programmed software modules for controlling the processors 100, and/or other programmable circuits or logic units such as ASICs (application specific integrated circuits) or the likes. In an embodiment, a functional part of the electronic circuit 10 is arranged in a separate housing and implements a further programmable control device 10*, e.g. a computer system, comprising e.g. one or more processors 100* with program and data memory and programmed software modules for controlling the processors 100*, and/or other programmable circuits or logic units such as ASICs or the like. Reference numeral 17 refers to a communication link enabling communication between the processors 100, 100* or other programmable circuits or logic units of the electronic circuit 10. Depending on the embodiment and/or configuration, the communication link 17 comprises one or more electrical connections, an electronic bus, a wired communication network, and/or a wireless communication network.

The laser source 11 comprises a femtosecond laser for producing femtosecond laser pulses, which have pulse widths of typically 10 fs to 1000 fs (1 fs = 10⁻¹⁵ s). The laser source 11 is arranged in a separate housing or in a housing shared with the focusing optical module 12.

The focusing optical module 12 is configured to focus the pulsed laser beam B or the laser pulses, respectively, onto a focal spot S in or on the eye tissue, i.e. for making the pulsed laser beam B converge to a focus or focal spot in or on the eye tissue. The focusing optical module 12 comprises one or more optical lenses. In an embodiment, the focusing optical module 12 comprises a focus adjustment device for setting the focal depth of the focal spot S, for example one or more movable lenses, in the focusing optical module 12 or upstream of the focusing optical module 12, or a drive for moving the entire focusing optical module 12 along the projection axis p (z-axis). By way of example, the focusing optical module 12 is installed in an application head 14, which can be placed onto the eye 2. The person skilled in the art will understand that in cases where the focusing optical module 12 is adjusted (focus) or moved as part of the scanning process or scanning actuation, the focusing optical module 12 and associated drives (actuators) 1310 can be viewed and considered as parts of the scanner system 13, implementing a z-scanner 131 configured to modulate the depth z of the focal spot S along the projection axis p.

As illustrated schematically in Figure 1, the ophthalmological device 1 comprises a patient interface 18 for attaching the application head 14 or the focusing optical module 12, respectively, onto the eye 2. Depending on the embodiment, the patient interface 18 is connected to the application head 14 in a fixed or removable manner. The patient interface 18 comprises an optional contact body 15 and one or more suction elements configured to fix the contact body 15 and thus the patient interface 18 to the cornea 20. For example, the one or more suction elements are arranged in a fastening ring 16, e.g. a vacuum-controlled suction ring, whereby the one or more suction elements are connected fluidically to a suction pump. The contact body 15, also referred to as applanation body, is at least partly light-transparent. In the state where the patient interface 18 or the contact body 15, respectively, is fixed to the cornea 20, the cornea 20 is applanated where the contact body 15 is in contact with the exterior (anterior) surface of the cornea 20.

In an embodiment, the ophthalmological device 1 further comprises a measurement system 19 configured to determine positional reference data of the cornea 20. Depending on the embodiment, the measurement system 19 comprises a video capturing system, an optical coherence tomography (OCT) system, and/or a structured light illumination system. Accordingly, the measurement data or positional reference data determined by the measurement system 19 includes video data, including top view data (comprising two-dimensional images), and/or OCT data of the cornea 20 (comprising three-dimensional tomography data). The measurement system 19 is configured to determine the positional reference data of the cornea 20 also in an applanated state of the cornea 20. The measurement system 19 is connected to and/or integrated with the electronic circuit 10 which is further configured to control the scanner system 13, using the positional reference data from the measurement system 19.

Aside from the optional positional reference data from the measurement system 19, the electronic circuit 10 is configured to use treatment control data to control the scanner system 13 to move the focal spot S in or on the eye tissue to target locations along a processing path t defined by the treatment control data to process the curved treatment face C in and/or on the eye tissue. The treatment control data is stored in the data memory and/or data store of the electronic circuit 10 and/or received via a communication link 17 from a separate computer system.

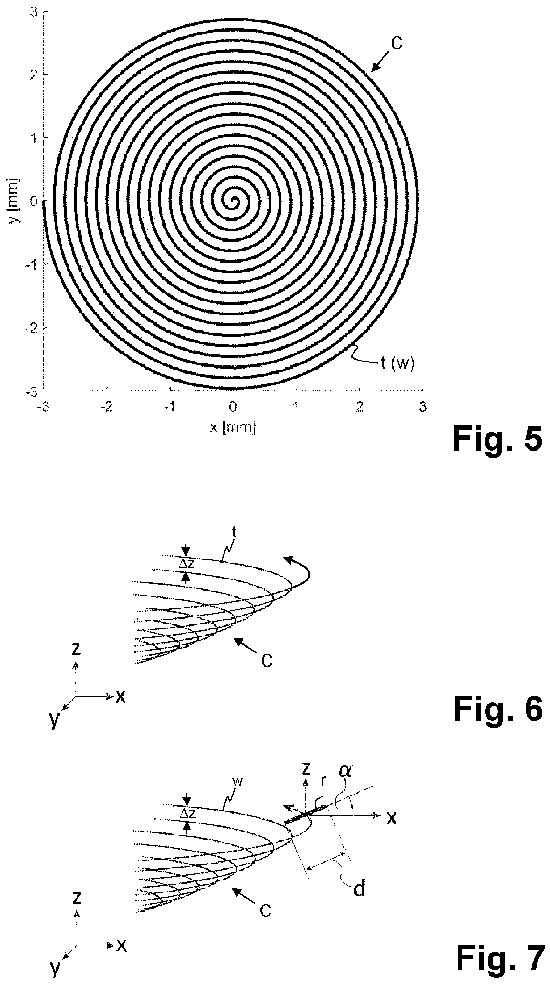

Essentially, the treatment control data defines the treatment pattern with the processing path t along which the scanner system 13 is to move the focal spot S for processing the curved treatment face C in the eye tissue. In effect, the treatment control data or the processing path t, respectively, define a sequence of consecutive target locations in a three-dimensional x/y/z-space for processing a three-dimensional curved treatment face C in eye tissue. Depending on the embodiment and/or configuration, for defining the processing path t, the treatment control data comprises path definition data, e.g. coordinates, positions, positional references, etc., and/or path processing data, e.g. instructions, operations, and/or procedures for the scanner system 13 and its components, described below in more detail. As illustrated schematically in Figures 5, 6, 8 or 9, the processing path t can be defined as a simple trajectory for the focal spot S to be moved along by the scanner system 13 for processing the curved treatment face C; or, as illustrated schematically in Figure 7, the processing path t can be defined as a composite trajectory, e.g. comprising a feed line w and a scan line r, extending transversely with respect to the feed line w, for the focal spot S to be moved along by the scanner system 13 for processing the curved treatment face C. The treatment control data comprises further control data for controlling the laser source 11 and the scanner system 13, e.g. processing speed, pulse rate, pulse energy, etc.

Processing the curved treatment face C imposes scanning requirements that must be met by a scanner system 13. More specifically, the scanning requirements are determined by the dynamics involved in moving the focal spot S along the processing path t, defined by the treatment control data, for processing the curved treatment face C. The dynamics include the speed with which a focal spot is to be placed and moved along the processing path and the distances that must be covered by the focal spot within a given time, at a defined speed, with a defined acceleration, and/or with a defined speed of acceleration (jerk/jump). The scanning requirements include a depth scanning requirement, representing the required modulation of the depth z of the focal spot S when moved along the processing path t defined by the treatment control data. The depth scanning requirement includes the dynamics required for the modulation of the depth z of the focal spot S along the processing path t defined by the treatment control data. The required depth scanning dynamics comprise the required depth scanning speed, the required depth scanning frequency, the required amplitude of depth modulation at a particular speed of the depth modulation, the required amplitude of depth modulation at a particular frequency of the depth modulation, the required acceleration of the depth modulation, and/or the required speed of the acceleration of the depth modulation.

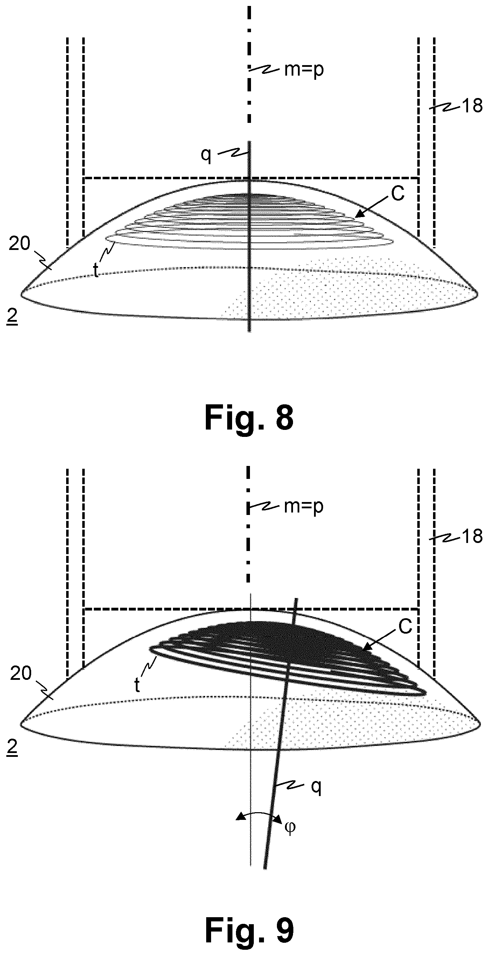

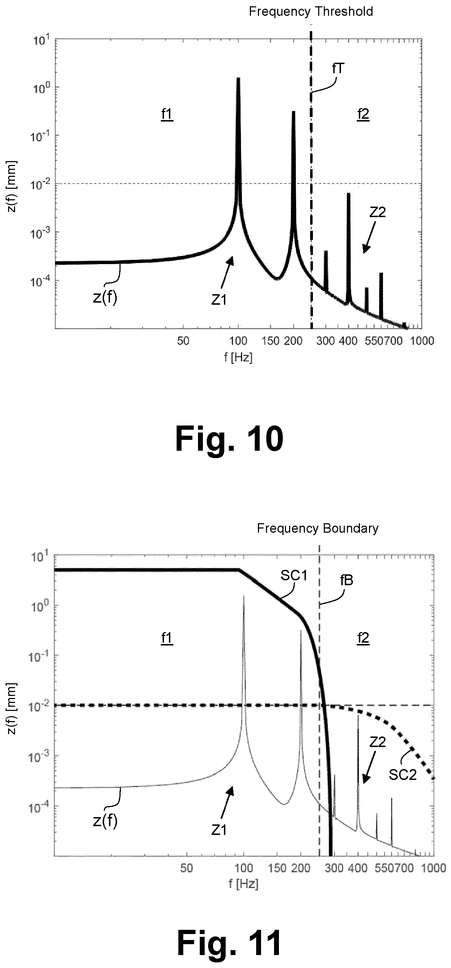

Figures 10 and 11 illustrate an example of the depth scanning requirement of moving the focal spot S along the processing path t for processing a curved treatment face C with a rotational asymmetry (astigmatic), e.g. caused by an irregular (bumpy, dented) curved treatment face C. It is pointed out that, depending on the scenario, Figures 10 and 11 illustrate the depth scanning requirement of moving the focal spot S along the complete processing path t or along only a part of the processing path t for processing the curved treatment face C. Rotational asymmetry of the curved treatment face C may also be a result of rearranging a planned rotationally symmetric curved treatment face C as an adjustment to a tilt of the eye 2 with respect to the patient interface 18 or vice versa, as illustrated schematically in Figure 9. In such a scenario, the central axis q of the eye 2 is not aligned with the optical axis of the focusing optical module 12 and/or the central axis m of the patient interface 18, respectively, but is transverse to the optical axis and/or the central axis m with a tilting angle φ. Correspondingly, the central axis of the curved treatment face C, i.e. the treatment pattern with the processing path t for processing the curved treatment face C, as defined by the treatment control data, is not aligned with the optical axis of the focusing optical module 12 and/or the central axis m of the patient interface 18, but is transverse to the optical axis and/or the central axis m with a tilting angle φ. Accordingly, in the tilted eye 2 shown in Figure 9, the curved treatment face C, which is to be positioned and processed in the eye tissue as illustrated in Figure 8 for the non-tilted eye 2, and correspondingly the processing path t are rearranged with respect to the focusing optical module 12 or the patient interface 18, as illustrated in Figure 9.

The scanner system 13 is configured to move the focal spot S to target locations in or on the eye tissue by guiding and directing the pulsed laser beam B and thus the focal spot S to target locations in or on the eye tissue.

As illustrated schematically in Figure 1, the scanner system 13 comprises two cascaded z-scanners 131, 132, configured to modulate the depth z of the focal spot S along the projection axis p, and an x/y-scanner system 133, configured to move the focal spot S in directions normal to the projection axis p. It is pointed out that the sequential arrangement of the devices and components of the scanner system 13 may be different from the arrangement illustrated schematically in Figure 1. For example, z-scanner 132 may be arranged downstream of the z-scanner 131, or at least one of the z-scanners 131, 132 may be arranged downstream of the x/y-scanner 133.

As illustrated schematically in Figure 1, the first z-scanner 131 comprises an actuator 1310 configured to actuate optical elements, e.g. on or more optical lenses, e.g. one or more optical lenses of the optical module 12, for modulating the depth z of the focal spot S along the projection axis p with first scan performance characteristics. The second z-scanner 132 comprises an actuator 1320 configured to actuate optical elements, e.g. one or more optical lenses, e.g. arranged upstream of the focusing optical module 12, for modulating the depth z of the focal spot S along the projection axis p with second scan performance characteristics. The scan performance characteristics indicate the dynamic depth scanning capabilities of the respective z-scanner 131, 132. The scan performance characteristics or the dynamic depth scanning capabilities, respectively, include a maximum depth scanning speed, a maximum depth scanning frequency, a maximum amplitude of depth modulation at a particular speed or frequency of the depth modulation, a maximum acceleration of the depth modulation, and/or a maximum speed of the acceleration of the depth modulation. For the sake of clarity, it is pointed out that the dynamic depth scanning capabilities are to be understood with reference and respect to the eye 2, for example, a maximum amplitude of depth modulation refers to the actual movement of the focal spot in the eye 2 rather than a movement or displacement of optical scanner components arranged remote from the eye. The dynamic depth scanning capabilities of the second z-scanner 132 are greater than the dynamic depth scanning capabilities of the first z-scanner 131. More specifically, the maximum depth scanning speed or frequency of the second z-scanner 132 is faster than the maximum depth scanning speed or frequency of the first z-scanner 131; the maximum acceleration of the depth modulation of the second z-scanner 132 is greater than the maximum acceleration of the depth modulation of the first z-scanner 131; and the maximum speed of the acceleration of the depth modulation of the second z-scanner 132 is greater than the maximum speed of the acceleration of the depth modulation of the first z-scanner 131; while the maximum amplitude of depth modulation of the first z-scanner 131 is greater than the maximum amplitude of depth modulation of the second z-scanner 132. With regards to different dynamic performance ranges, where a comparatively lower dynamic performance range is characterized by comparatively lower depth scanning speed or frequency, and a comparatively higher dynamic performance range is characterized by comparatively higher depth scanning speed or frequency, the maximum amplitude of depth modulation of the second z-scanner 132 is lower than the maximum amplitude of depth modulation of the first z-scanner 131 in the comparatively lower dynamic performance range, whereas the maximum amplitude of depth modulation of the second z-scanner 132 is greater than the maximum amplitude of depth modulation of the first z-scanner 131 in the comparatively higher dynamic performance range. Figure 11 illustrates examples of the scan performance characteristics with the dynamic depth scanning capabilities SC1 of the first z-scanner 131 and the dynamic depth scanning capabilities SC2 of the second z-scanner 132. Figure 11 shows with a double-logarithmic scale on the horizontal axis the frequency f (or speed) of the actuation in Hz, and on the vertical axis the amplitude or distance z(f) in mm actuated at the respective frequency f. Depending on the embodiment or configuration, the actuated amplitude or distance z(f) indicates the actual amplitude or distance of the movement of the actuated optics or the amplitude or distance Δz of the movement of focus caused by this actuation of the optics. In the example of Figure 11, the dynamic depth scanning capabilities SC1 of the first z-scanner 131 are characterized by a maximum amplitude or distance of actuation of 5mm up to a scanning speed or frequency of approximately 100Hz, with a decrease of the amplitude or distance of actuation for scanning speeds or frequencies above 100Hz. In the example of Figure 11, the dynamic depth scanning capabilities SC2 of the second z-scanner 132 are characterized by a maximum amplitude or distance of actuation of the first z-scanner 131 of 0.01mm up to a scanning speed or frequency of approximately 300Hz, with a decrease of the amplitude or distance of actuation for scanning speeds or frequencies above 300Hz. In this example, where the z-scanners 131, 132 are oscillating, e.g. sinusoidally, the scanning speed depends linearly on the scanning frequency. This case is representative for spiral shaped feed lines w, for example. Accordingly, in such cases, the terms "scanning speed" and "scanning frequency" can be used synonymously. In other scenarios, the interdependency of scanning speed and scanning frequency, and thus maximum scanning speed and maximum scanning frequency, is more complex and, therefore, scanning speed and scanning frequency need to be treated differently.

The x/y-scanner system 133 comprises one or more scanner devices 1331, 1332, also referred to as slow scanner devices, configured to guide and direct the pulsed laser beam B and thus the focal spot S along processing the path t or a feed line w thereof, e.g. a spiral shaped feed line w, in a x/y-work-plane which is normal to the z-axis, whereby the z-axis is aligned with or essentially parallel to the projection axis p of the focusing optical module 12, as illustrated schematically in Figure 1. Depending on the embodiment, the one or more scanner devices 1331, 1332 comprise one or more actuators configured to move the focusing optical module 12 such that the focal spot S is moved along the processing path t or the feed line w in the x/y-work-plane, and/or one or more deflection mirrors, for example, each movable about one or two axes, configured to deflect the pulsed laser beam B and/or the laser pulses such that the focal spot S is moved along the processing path t or the feed line w in the x/y-work-plane.

The electronic circuit 10 is configured to control in a synchronized fashion the x/y-scanner system 133 and the z-scanners 131, 132 to move the focal spot S along a processing path t or a feed line w in the three-dimensional x/y/z-space, e.g. a spiral shaped processing path t or feed line w in the three-dimensional x/y/z-space. Figure 5 illustrates schematically in top view a spiral shaped processing path t or feed line w for processing the curved treatment face C, e.g. in the cornea 20. Figure 6 shows a schematic three-dimensional view of a section of a spiral shaped processing path t or feed line w for processing the curved treatment face C, e.g. in the cornea 20.

In an embodiment with a composite processing path t, the scanner system 13 comprises one or more further scanner devices 130, also referred to as fast scanner devices, configured to guide and direct the pulsed laser beam B and thus the focal spot S along a scan line r at a scanning speed that is comparatively faster than the scanning speed of the aforementioned slow scanner devices 1331, 1332. For example, the fast scanner device 130 comprises a polygon scanner or a resonant scanner. The fast scanner device 130 is configured to move the focal spot S, overlaid on the movement along the feed line w, along a scan line r that runs transversal to the feed line w, in other words, it runs through the feed line w, at an angle to the feed line w, as illustrated in Figure 7. The electronic circuit 10 is configured to control the x/y-scanner system 133, the z-scanners 131, 132, and the fast scanner device 130 in a synchronized fashion. The electronic circuit 10 controls scanner parameters such as scan speed or frequency, scan amplitude, and/or scan direction, angle or orientation (tilt).

As illustrated schematically in Figure 7, in an embodiment, a further divergence-modulator is synchronized with the fast scanner device 130 to effect a movement of the focal spot S along a scan line r which is bent and/or tilted with a tilting angle α from the x/y-plane.

In an embodiment, the scanner system 13 further comprises an optional length modulator 134 configured to modulate the length d of the scan line r. For example, the length modulator 134 comprises an adjustable shutter device arranged downstream of the fast scanner device 130. For example, the length d of the scan line r is adjusted by controlling the length modulator 134, e.g. the shutter device, to let through a set number of laser pulses from the fast scanner device 130 for producing a corresponding number of focal spots S. The electronic circuit 10 is configured to control the length modulator 134 to adjust the length d of the scan line r with respect to the shape of the curved treatment face C to be processed, as illustrated schematically in Figure 7.

The synchronized combination of the movement of the focal spots S along the feed line w in the x/y/z-space by the x/y-scanner system 133 and the z-scanners 131, 132, with the overlaid movement of the focal spots S along the scan line r by the fast scanner device 130, and the tilting of the scan line r with a tilting angle α from the x/y-plane by the z-scanners 131, 132 or the further divergence-modulator, and optionally the adjustment of the length d of the scan line r by the length modulator 134, as illustrated in Figure 7, makes it possible not only to process a curved treatment face C inside the eye tissue, e.g. to generate curved incision faces inside the cornea 20, but also to perform with great flexibility volumetric treatment of the eye tissue, e.g. volumetric ablation of corneal tissue. For example, volumetric treatment is achieved inside the eye tissue by driving the scan line r overlaid on the feed line w with a continuous increase Δz in z-direction (e.g. per cycle of a spiral shaped feed line w) to generate superposed treatment layers with partially overlapping focal spots S along the processing path t, feed line w, and scan line r (as illustrated in Figures 2 to 4), among neighbouring segments of the processing path t or the feed line w, and neighbouring scan lines r (as illustrated in Figures 2 and 4), and among adjacent superposed treatment layers or segments of the processing path t or the feed line w, and neighbouring scan lines r, respectively (as illustrated in Figures 3 and 4). Various further and more specific embodiments of the scanner system 13 are described by the applicant in patent applications US 2019/0015250, US 2019/0015251, and US 2019/0015253.

For moving the focal spot S along the processing path t to process the curved treatment face C, the electronic circuit 10 controls in a synchronized fashion the actuation performed by all the systems, devices, modules, and components of the scanner system 13.

The electronic circuit 10 is further configured to determine from the treatment control data individual depth scanning components z1, z2 for the z-scanners 131, 132. Specifically, the electronic circuit 10 is configured to determine from the treatment control data the depth scanning requirement, i.e. the modulation of the depth z of the focal spot S required when being moved along the processing path t defined by the treatment control data, and to divide the depth scanning requirement into individual depth scanning components z1, z2 for the z-scanners 131, 132. More specifically, the electronic circuit 10 is configured to determine the individual depth scanning components z1, z2 for the z-scanners 131, 132 taking into consideration the scan performance characteristics of the z-scanners 131, 132 with their respective dynamic depth scanning capabilities.

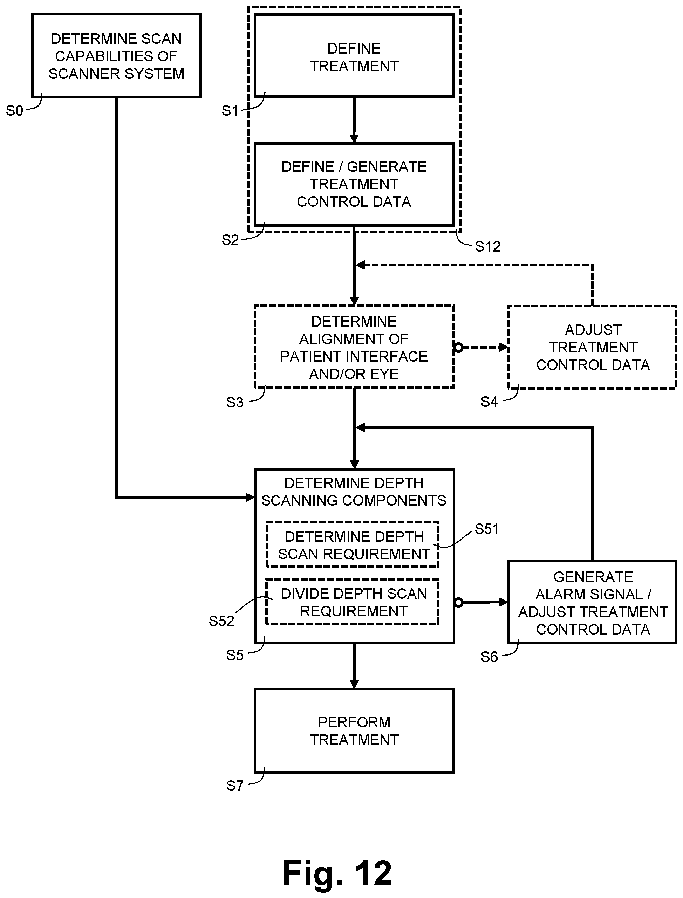

In the following paragraphs, described with reference to Figure 12 are possible steps performed by the electronic circuit 10 for controlling the ophthalmological device 1, specifically its laser source 11 and scanner system 13, for processing in eye tissue a curved treatment face C defined by treatment control data, and particularly for determining individual depth scanning components z1, z2 for the z-scanners 131, 132 to process the curved treatment face C.

In preparatory step S0, the electronic circuit 10 determines the scan capabilities of the scanner system 13. Particularly, in preparatory step S0, the electronic circuit 10 determines the scan performance characteristics SC1 of the first z-scanner 131, indicating the dynamic depth scanning capabilities of the first z-scanner 131, and the scan performance characteristics SC2 of the second z-scanner 132, indicating the dynamic depth scanning capabilities of the second z-scanner 132. Depending on embodiment and/or configuration, the electronic circuit 10 determines the scan capabilities, including the scan performance characteristics SC1, SC2 of the z-scanners 131, 132, by loading scanner characteristics data, e.g. as provided by the manufacturer of the scanners, or as previously measured and recorded during a performance test, or by running an actual performance test to operate the scanner system 13 in a test mode (test routine) and measure and record the scan capabilities of the scanner system 13. The scanner characteristics data, loaded and/or measured, indicates the scan capabilities of the scanner system 13, particularly the scan performance characteristics SC1, SC2 of the z-scanners 131, 132 with their respective dynamic depth scanning capabilities.

In further preparatory step S1, the electronic circuit 10 determines the treatment to be performed by the ophthalmological device 1. Depending on embodiment and/or configuration, the electronic circuit 10 determines the treatment by receiving, from a separate computer or from a user via a user interface, a selection of a predefined treatment, or by receiving treatment definitions from the user via a computer aided design (CAD) software application.

In step S2, for the treatment defined in step S1, the electronic circuit 10 determines the treatment control data for controlling the scanner system 13 to process the curved treatment face C in the eye tissue. The electronic circuit 10 determines the treatment control data for the selected treatment and/or treatment definitions received in step S1. The person skilled in the art will understand that steps S1 and S2 can be executed by the electronic circuit 10 as a combined step S12, whereby the electronic circuit 10 receives selections and definitions of a treatment and generates the treatment control data for the received selections and definitions of the treatment.

In optional step S3, the electronic circuit 10 determines the actual alignment (in situ) of the focussing optical module 12 and/or the patient interface 18 with respect to the eye 2. The actual alignment is determined in situ when the patient interface 18 is attached to the eye 2 and the focussing optical module 12 is thereby fixed on the eye 1. More specifically, the electronic circuit 10 determines the alignment of the central axis m of the patient interface 18 and the central axis q of the eye 2. In an embodiment, the electronic circuit 10 uses positional reference data from the measurement system 19 to determine the alignment.

In case there is a tilting angle φ in the alignment of the patient interface 18 and the eye 2, i.e. a tilting angle φ between the central axis m of the patient interface 18 and the central axis q of the eye 2, the electronic circuit 10 continues processing in optional step S4; otherwise, if the patient interface 18 and the eye 2 are aligned, processing continues in step S5.

In optional step S4, in case there is a tilting angle φ, the curved treatment face C to be processed in the eye tissue is to be tilted accordingly, with the same tilting angle φ between the central axis m of the patient interface 18 and the central axis of the curved treatment face C (see Figure 9). Thus, in optional step S4, the electronic circuit 10 adjusts the treatment control data for controlling the scanner system 13 to process the curved treatment face C in the eye tissue with a tilting angle φ with respect to the central axis m of the patient interface 18.

In step S5, the electronic circuit 10 determines the depth scanning components z1, z2 for the z-scanners 131, 132.

In step S51, the electronic circuit 10 determines the depth scan requirement for processing the curved treatment face C, as defined by the treatment control data. More specifically, the electronic circuit 10 determines the required dynamics for modulating the depth z of the focal spot S along the processing path t defined by the treatment control data. In the example illustrated in Figures 10 and 11, the electronic circuit 10 determines the amplitude of depth modulation z(f) depending on the frequency f of the depth modulation, as required for moving the focal spot S along the processing path t for processing the curved treatment face C defined by the treatment control data.

In step S52, the electronic circuit 10 divides the depth scanning requirement into a first depth scanning component z1 for the first z-scanner 131 and a second depth scanning component z2 for the second z-scanner 132. The electronic circuit 10 uses the scan performance characteristics SC1, SC2 of the z-scanners 131, 132, with their respective dynamic depth scanning capabilities, to divide the depth scanning requirement into the depth scanning components z1, z2 for the z-scanners 131, 132. The electronic circuit 10 further uses the depth scan requirement, determined for the curved treatment face C, particularly, the required dynamics of the depth modulation z(f), to determine the depth scanning components z1, z2 for the z-scanners 131, 132.

In an embodiment, the electronic circuit 10 determines a phase difference between actuation by the first actuator 1310 of the first z-scanner 131 and actuation by the second actuator 1320 of the second z-scanner 132. For example, the phase difference between actuation by the actuators 1310, 1320 of the z-scanners 131, 132 is determined during system calibration and/or on a periodical basis and stored in a data storage of the electronic circuit 10. The electronic circuit 10 uses this actuation phase difference for generating the control signal for the first actuator 1310 to execute the first depth scanning component z1, and for generating the control signal for the second actuator 1320 to execute the second depth scanning component z2, so that the combined depth modulation effected by the z-scanners 131, 132 corresponds to the total depth scanning requirement required when the focal spot S is moved along the processing path t defined by the treatment control data, while the depth modulation effected by the z-scanners 131, 132 in opposite direction is avoided or at least minimized.

In the example illustrated in Figure 11, the electronic circuit 10 compares the performance characteristics SC1, SC2 of the z-scanners 131, 132 to the depth scanning requirement of the curved treatment face C, indicating the required depth modulation z(f). Based on this comparison, the electronic circuit 10 determines a frequency boundary fB which divides the depth scanning requirement into a first frequency range f1, where the required depth modulation z(f) is met and can be provided by the performance characteristics SC1 and dynamic depth scanning capabilities of the first z-scanner 131, and a second frequency range f2, where the required depth modulation z(f) is met and can be provided by the performance characteristics SC2 and dynamic depth scanning capabilities of the second z-scanner 132.

In an embodiment, the electronic circuit 10 determines from the treatment control data a spherical component of the curved treatment face C and a complementary component for the curved treatment face C, which complementary component complements the spherical component to make up the curved treatment face C. For example, the spherical component of the curved treatment face C corresponds to a spherical component of a refractive correction whereas the complementary component of the curved treatment face C corresponds to correction of higher order aberrations. Subsequently, the electronic circuit divides the depth scanning requirement into the first depth scanning component z1, as required for the modulation of the depth z of the focal spot S to move the focal spot S along a processing path t for processing the spherical component, and into the second depth scanning component z2, as required for the modulation of the depth z of the focal spot S to move the focal spot S along the processing path t for processing the complementary component.

The electronic circuit 10 performs a feasibility check by determining whether or not the scan requirements needed for processing the curved treatment face C, as defined by the treatment control data generated in step S2 and/or adjusted in steps S4 or S6, exceed the scan capabilities of the scanner system 13. Particularly, the electronic circuit 10 determines the depth scanning feasibility by checking whether the depth scanning requirement is achievable with the required dynamics, without exceeding the performance characteristics SC1 with the dynamic depth scanning capabilities of the first z-scanner 131 or the performance characteristics SC2 with the dynamic depth scanning capabilities of the second z-scanner 132. In case of a negative outcome of the feasibility check, which indicates that moving the focal spot S along the processing path t, defined by the treatment control data, exceeds the scan capabilities of the scanner system 13, the electronic circuit 10 continues processing in step S6. Particularly, if the depth scanning requirement cannot be met with the performance characteristics SC1, SC2 of the z-scanners 131, 132, the electronic circuit 10 proceeds in step S6.

Depending on the embodiment and/or selected configuration, performing the feasibility check includes the electronic circuit 10 computing and executing a simulation or a "dry run" of moving the focal spot S along the processing path t defined by the treatment control data.

In the case of the computer simulation of moving the focal spot S along the processing path t, the electronic circuit 1 simulates the movement of the focal spot S using the treatment control data and the determined depth scanning components z1, z2 to drive a computer model and/or simulation algorithms of the scanner system 13, particularly of the of the z-scanners 131, 132, whereby the computer model uses the scan capabilities of the scanner system 13, particularly the performance characteristics SC1, SC2 of the z-scanners 131, 132.

In case of the "dry run" of moving the focal spot S along the processing path t, the electronic circuit 1 sets the laser source 11 to a deactivated state or a reduced energy level, without any (lasting) effect to the eye tissue, and then uses the treatment control data and the determined depth scanning components z1, z2 to control the scanner system 13, particularly the z-scanners 131, 132, to move the focal spot S or the virtual or imaginary focal spot, respectively, along the processing path t, defined by the treatment control data, to perform a "dry run" of processing the curved treatment face C in the eye tissue.

In both cases, the electronic circuit 10 determines the scan requirements, particularly the depth scanning requirement, used for processing the curved treatment face C defined by the treatment control data. In other words, the electronic circuit 10 determines the scan dynamics required of the scanner system 13, particularly of the z-scanners 131, 132, for moving the focal spot S along the processing path t, defined by the treatment control data.

For assessing feasibility, the electronic circuit 10 checks for the scanner system 13, particularly of the z-scanners 131, 132, whether the required scan dynamics for moving the focal spot S along the processing path t, defined by the treatment control data, exceed the performance characteristics the scanner system 13, particularly of the performance characteristics SC1, SC2 of the z-scanners 131, 132.

In step S6, the electronic circuit 10 generates an alarm signal and/or adjusts the treatment control data. The alarm signal comprises, an acoustic, an optical, and/or an electronic signal, the latter being usable for triggering and initiating emergency measures in an external system. The alarm signal may further comprise an error message for the operator, e.g. indicating the cause or reason for the failed plausibility check and/or indicating possible measures for improving plausibility. For example, the alarm signal may signal to the operator that restarting the procedure with a different position and/or alignment of the patient interface 18 is necessary to proceed. Adjusting the treatment control data comprises the electronic circuit 10 changing the treatment control data such that the scan requirements, particularly the depth scanning requirement, no longer exceed the scan capabilities of the scanner system 13, particularly the performance characteristics SC1, SC2 of the z-scanners 131, 132. For example, the electronic circuit 10 reduces or varies the speed of moving the focal spot S along the processing path t, to avoid that moving the focal spot S along the processing path t, defined by the treatment control data, exceeds the scan capabilities of the scanner system 13, particularly the performance characteristics SC1, SC2 of the z-scanners 131, 132. Alternatively, the treatment control data is adjusted by altering the processing path t, e.g. its shape, to reduce the scan requirements for a modified curved treatment face C. In addition or alternatively, the electronic circuit 10 alters the processing path t for a modified curved treatment face C, which differs from the initial curved treatment face, defined by the initial treatment control data, only by a maximum deviation threshold, as defined by numerical or ophthalmological criteria, such as a threshold of reduced refractive correction. As illustrated in Figure 12, subsequently to adjusting the treatment control data, the electronic circuit 10 continues processing in step S5, re-determining the depth scanning components, verifying that the scan requirements, particularly the depth scanning requirement, needed for processing the curved treatment face C, as defined by the adjusted treatment control data, do not exceed the scan capabilities of the scanner system 13, particularly the performance characteristics SC1, SC2 of the z-scanners 131, 132.

In case of a positive outcome of the feasibility check, which indicates that moving the focal spot S along the processing path t, defined by the treatment control data, does not exceed the scan capabilities of the scanner system 13, the electronic circuit 10 continues processing in step S7.

In step S7, the electronic circuit 10 uses the treatment control data or the adjusted treatment control data, respectively, for controlling the scanner system 13 to move the focal spot S along the processing path t defined by the treatment control data for processing the curved treatment face C in the eye tissue.

## Claims

1. An ophthalmological device (1) for processing a curved treatment face (C) in eye tissue, the ophthalmological device (1) comprising:
a laser source (11) configured to generate a pulsed laser beam (B);
a focussing optical module (12) having a projection axis (z) and being configured to make the pulsed laser beam (B) converge onto a focal spot (S) in or on the eye tissue;
a scanner system (13) comprising a first z-scanner (131) configured to modulate a depth (z) of the focal spot (S) along the projection axis (p) with first scan performance characteristics, indicating dynamic depth scanning capabilities of the first z-scanner (131), a second z-scanner (132) configured to modulate the depth (z) of the focal spot (S) along the projection axis (p) with second scan performance characteristics, indicating dynamic depth scanning capabilities of the second z-scanner (132) which are greater than the dynamic depth scanning capabilities of the first z-scanner (131), and an x/y-scanner system (133) configured to move the focal spot (S) in directions normal to the projection axis (p); and
a circuit (10) configured to control the scanner system (13) to move the focal spot (S) to target locations on the curved treatment face (C) along a processing path (t) defined by treatment control data,
**characterized in that** the circuit is further configured:
to determine from the treatment control data a depth scanning requirement, representing modulation of the depth (z) of the focal spot (S) along the processing path (t) defined by the treatment control data,
to divide the depth scanning requirement into a first depth scanning component (z1) for the first z-scanner (131) and a second depth scanning component (z2) for the second z-scanner (132),
to control the first z-scanner (131) using the first depth scanning component (z1), and
to control the second z-scanner (132) using the second depth scanning component (z2).

2. The ophthalmological device (1) of claim 1, wherein the circuit (10) is configured to determine the first depth scanning component (z1) and the second depth scanning component (z2), using the first scan performance characteristics and the second scan performance characteristics.

3. The ophthalmological device (1) of claim 1, wherein the circuit (10) is configured to determine from the treatment control data a spherical component of the curved treatment face (C) and a complementary component for the curved treatment face (C), the complementary component complementing the spherical component to make up the curved treatment face (C), and to divide the depth scanning requirement into the first depth scanning component (z1), as required for the modulation of the depth (z) of the focal spot (S) for the spherical component, and the second depth scanning component (z2), as required for the modulation of the depth (z) of the focal spot (S) for the complementary component.

4. The ophthalmological device (1) of claim 1, wherein determining the depth scanning requirement includes determining required dynamics of the modulation of the depth (z) of the focal spot (S) along the processing path (t) defined by the treatment control data; and the circuit (10) is configured to determine the first depth scanning component (z1) using the first scan performance characteristics and the required dynamics, and to determine the second depth scanning component (z2) using the second scan performance characteristics and the required dynamics.

5. The ophthalmological device (1) of claim 4, wherein the required dynamics comprise at least one of: a required depth scanning speed, a required depth scanning frequency, a required amplitude of depth modulation at a particular speed of the depth modulation, a required amplitude of depth modulation at a particular frequency of the depth modulation, a required acceleration of the depth modulation, or a required speed of the acceleration of the depth modulation.

6. The ophthalmological device (1) of one of claims 4 or 5, wherein the circuit (10) is configured to determine depth scanning feasibility by checking whether the depth scanning requirement is achievable for the required dynamics, without exceeding at least one of the dynamic depth scanning capabilities of the first z-scanner (131) or the dynamic depth scanning capabilities of the second z-scanner (132), and to adjust the treatment control data to reduce or vary a speed of moving the focal spot (S) along the processing path (t), in case the depth scanning feasibility is not affirmed.

7. The ophthalmological device (1) of claim 6, wherein the circuit (10) is configured to determine the depth scanning feasibility by computing a simulation of moving the focal spot (S) along the processing path (t), defined by the treatment control data, using the first depth scanning component (z1) and the second depth scanning component (z2).

8. The ophthalmological device (1) of one of claims 6 or 7, wherein the circuit (10) is configured to perform the depth scanning feasibility by controlling the scanner system (13) to move the focal spot (S) along the processing path (t), defined by the treatment control data, using the first depth scanning component (z1) and the second depth scanning component (z2), while setting the laser source (11) to at least one of: a deactivated state or a reduced energy without any effect to the eye tissue.

9. The ophthalmological device (1) of one of claims 1 to 8, wherein the first scan performance characteristics include a first maximum depth scanning speed or frequency; the second scan performance characteristics include a second maximum depth scanning speed or frequency which is faster than the first maximum depth scanning speed or frequency; and the circuit (10) is configured to determine the first depth scanning component (z1) using the first maximum depth scanning speed or frequency, and to determine the second depth scanning component (z2) using the a second maximum depth scanning speed or frequency.

10. The ophthalmological device (1) of one of claims 1 to 9, wherein the first scan performance characteristics include a first maximum amplitude of depth modulation at a particular speed or frequency of the depth modulation; the second scan performance characteristics include a second maximum amplitude of depth modulation at the particular speed or frequency of the depth modulation, the second maximum amplitude of depth modulation being smaller than the first maximum amplitude of depth modulation in a comparatively lower dynamic performance range, and the second maximum amplitude of depth modulation being greater than the first maximum amplitude of depth modulation in a comparatively higher dynamic performance range; and the circuit (10) is configured to determine the first depth scanning component (z1) using the first maximum amplitude of depth modulation, and to determine the second depth scanning component (z2) using the second maximum amplitude of depth modulation.

11. The ophthalmological device (1) of one of claims 1 to 10, wherein the first scan performance characteristics include at least one of: a first maximum acceleration of the depth modulation, or a first maximum speed of the acceleration of the depth modulation; the second scan performance characteristics include at least one of: a second maximum acceleration of the depth modulation, greater than the first maximum acceleration of the depth modulation, or a second maximum speed of the acceleration of the depth modulation, greater than the first maximum speed of the acceleration of the depth modulation; and the circuit (10) is configured to determine the first depth scanning component (z1) using the first maximum acceleration or the first maximum speed of the acceleration, and to determine the second depth scanning component (z2) using the second maximum acceleration or the second maximum speed of the acceleration.

12. The ophthalmological device (1) of one of claims 1 to 11, wherein the ophthalmological device (1) further comprises a patient interface (18) having a central axis (m) and being configured to fix the focussing optical module (12) on the eye (2); and the circuit (10) is further configured, in case of a tilt of the eye (2) with respect to the central axis (m) of the patient interface (18), to adapt the treatment control data to tilt the curved treatment surface (C) corresponding to the tilt of the eye (2), prior to determining the depth scanning requirement, and to use the adapted treatment control data to determine the depth scanning requirement and divide the depth scanning requirement into the first depth scanning component (z1) and the second depth scanning component (z2).

13. The ophthalmological device (1) of one of claims 1 to 12, wherein the scanner system (13) is configured to move the focal spot (S) along a spiral-shaped processing path (t).

14. The ophthalmological device (1) of one of claims 1 to 13, wherein the x/y-scanner system (133) comprises a first x/y-scanner (1331), configured to move the focal spot (S) with a feed speed along a feed line (w) of the processing path (t), and the x/y-scanner system (133) comprises a second x/y-scanner (1332), configured to move the focal spot (S) with a scan speed, which is higher than the feed speed, along a scan line (r) extending transversely with respect to the feed line (w) of the processing path (t).

15. The ophthalmological device (1) of one of claims 1 to 14, wherein the first z-scanner (131) comprises a first actuator (1310), the second z-scanner (132) comprises a second actuator (1320), and the circuit (10) is configured to determine a phase difference between actuation by the first actuator (1310) and actuation by the second actuator (1320), and to generate, for the first depth scanning component (z1), a first control signal for the first actuator (1310) and, for the second depth scanning component (z2), a second control signal for the second actuator (1320), using the phase difference.

16. A computer program product comprising a non-transitory computer-readable medium having stored thereon computer program code for controlling a processor (100) of an ophthalmological device (1) which comprises a laser source (11) configured to generate a pulsed laser beam (B), a focussing optical module (12) having a projection axis (z) and being configured to make the pulsed laser beam (B) converge onto a focal spot (S) in the eye tissue, and a scanner system (13) comprising a first z-scanner (131), configured to modulate a depth (z) of the focal spot (S) along the projection axis (p) with first scan performance characteristics, indicating dynamic depth scanning capabilities of the first z-scanner (131), a second z-scanner (132), configured to modulate the depth (z) of the focal spot (S) along the projection axis (p) with second scan performance characteristics, indicating dynamic depth scanning capabilities of the second z-scanner (132) which are greater than the dynamic depth scanning capabilities of the first z-scanner (131), and an x/y-scanner system (133) configured to move the focal spot (S) in directions normal to the projection axis (p); whereby the computer program code is configured to control the processor (100) such that the processor (100):
uses treatment control data to control the scanner system (13) to move the focal spot (S) in the eye tissue to target locations along a processing path (t), defined by the treatment control data to process a curved treatment face (C) in the eye tissue;
**characterized in that** the computer program code is further configured to control the processor (100) such that the processor (100):
determines from the treatment control data a depth scanning requirement, representing modulation of the depth (z) of the focal spot (S) along the processing path (t) defined by the treatment control data;
divides the depth scanning requirement into a first depth scanning component (z1) for the first z-scanner (131) and a second depth scanning component (z2) for the second z-scanner (132);
controls the first z-scanner (131) using the first depth scanning component (z1); and
controls the second z-scanner (132) using the second depth scanning component (z2).

## Patentansprüche

1. Ophthalmologische Vorrichtung (1) zur Bearbeitung einer gekrümmten Behandlungsoberfläche (C) in Augengewebe, wobei die ophthalmologische Vorrichtung (1) umfasst:
eine Laserquelle (11), die zum Erzeugen eines gepulsten Laserstrahls (B) konfiguriert ist;
ein fokussierendes optisches Modul (12), das eine Projektionsachse (z) aufweist und dazu konfiguriert ist, den gepulsten Laserstrahl (B) auf einen Brennpunkt (S) in oder auf dem Augengewebe zu fokussieren;
ein Scannersystem (13), umfassend einen ersten z-Scanner (131), der dazu konfiguriert ist, eine Tiefe (z) des Brennpunkts (S) entlang der Projektionsachse (p) mit ersten Scan-Leistungsmerkmalen zu modulieren, welche die dynamische Tiefenscanfähigkeiten des ersten z-Scanners (131) angeben, und umfassend einen zweiten z-Scanner (132), der dazu konfiguriert ist, die Tiefe (z) des Brennpunkts (S) entlang der Projektionsachse (p) mit zweiten Scan-Leistungsmerkmalen zu modulieren, welche die dynamische Tiefenscanfähigkeiten des zweiten z-Scanners (132) angeben, die größer sind als die dynamischen Tiefenscanfähigkeiten des ersten z-Scanners (131), und ein x/y-Scannersystem (133), das dazu konfiguriert ist, den Brennpunkt (S) in Richtungen senkrecht zur Projektionsachse (p) zu bewegen; und
eine Schaltung (10), die dazu konfiguriert ist, das Scannersystem (13) so zu steuern, dass der Brennpunkt (S) entlang eines durch Behandlungsteuerungsdaten definierten Bearbeitungspfads (t) zu Zielorten auf der gekrümmten Behandlungsoberfläche (C) bewegt wird,
**dadurch gekennzeichnet, dass** der Schaltkreis ferner dazu konfiguriert ist:
aus den Behandlungssteuerungsdaten eine Tiefenscananforderung zu bestimmen, die eine Modulation der Tiefe (z) des Brennpunkts (S) entlang des durch die Behandlungssteuerungsdaten definierten Bearbeitungspfads (t) darstellt,
die Tiefenscananforderung in eine erste Tiefenscankomponente (z1) für den ersten z-Scanner (131) und eine zweite Tiefenscankomponente (z2) für den zweiten z-Scanner (132) zu unterteilen,
den ersten Z-Scanners (131) unter Verwendung der ersten Tiefenscankomponente (z1) zu steuern, und
den zweiten z-Scanners (132) unter Verwendung der zweiten Tiefenscankomponente (z2) zu steuern.

2. Ophthalmologische Vorrichtung (1) nach Anspruch 1, wobei die Schaltung (10) dazu konfiguriert ist, die erste Tiefenscankomponente (z1) und die zweite Tiefenscankomponente (z2) unter Verwendung der ersten Scan-Leistungsmerkmale und der zweiten Scan-Leistungsmerkmale zu bestimmen.

3. Ophthalmologische Vorrichtung (1) nach Anspruch 1, wobei die Schaltung (10) dazu konfiguriert ist, aus den Behandlungssteuerungsdaten eine sphärische Komponente der gekrümmten Behandlungsoberfläche (C) und eine komplementäre Komponente für die gekrümmte Behandlungsoberfläche (C) zu bestimmen, wobei die komplementäre Komponente die sphärische Komponente ergänzt, um die gekrümmte Behandlungsoberfläche (C) zu bilden, und die Tiefenscananforderung in die erste Tiefenscankomponente (z1), wie sie für die Modulation der Tiefe (z) des Brennpunkts (S) für die sphärische Komponente erforderlich ist, und die zweite Tiefenscankomponente (z2), wie sie für die Modulation der Tiefe (z) des Brennpunkts (S) für die komplementäre Komponente erforderlich ist, zu unterteilen.

4. Ophthalmologische Vorrichtung (1) nach Anspruch 1, wobei das Bestimmen der Tiefenscananforderung das Bestimmen der erforderlichen Dynamik der Modulation der Tiefe (z) des Brennpunkts (S) entlang des durch die Behandlungssteuerungsdaten definierten Bearbeitungspfads (t) umfasst; und wobei die Schaltung (10) dazu konfiguriert ist, die erste Tiefenscankomponente (z1) unter Verwendung der ersten Scan-Leistungsmerkmale und der erforderlichen Dynamik zu bestimmen, und um die zweite Tiefenscankomponente (z2) unter Verwendung der zweiten Scan-Leistungsmerkmale und der erforderlichen Dynamik zu bestimmen.

5. Ophthalmologische Vorrichtung (1) nach Anspruch 4, wobei die erforderliche Dynamik mindestens eines der folgenden Elemente umfasst: eine erforderliche Tiefenscangeschwindigkeit, eine erforderliche Tiefenscanfrequenz, eine erforderliche Amplitude der Tiefenmodulation bei einer bestimmten Geschwindigkeit der Tiefenmodulation, eine erforderliche Amplitude der Tiefenmodulation bei einer bestimmten Frequenz der Tiefenmodulation, eine erforderliche Beschleunigung der Tiefenmodulation, oder eine erforderliche Geschwindigkeit der Beschleunigung der Tiefenmodulation.

6. Ophthalmologische Vorrichtung (1) nach einem der Ansprüche 4 oder 5, wobei die Schaltung (10) dazu konfiguriert ist, die Durchführbarkeit des Tiefenscans zu bestimmen, indem sie prüft, ob die Tiefenscananforderung für die erforderliche Dynamik erreichbar ist, ohne mindestens eine der dynamischen Tiefenscanfähigkeiten des ersten Z-Scanners (131) oder der dynamischen Tiefenscanfähigkeiten des zweiten Z-Scanners (132) zu überschreiten, und dass sie die Behandlungssteuerungsdaten anpasst, um eine Geschwindigkeit der Bewegung des Brennpunkts (S) entlang des Bearbeitungspfads (t) zu reduzieren oder zu variieren, falls die Durchführbarkeit des Tiefenscans nicht bestätigt wird.

7. Ophthalmologische Vorrichtung (1) nach Anspruch 6, wobei die Schaltung (10) dazu konfiguriert ist, die Durchführbarkeit des Tiefenscans durch Berechnen einer Simulation der Bewegung des Brennpunkts (S) entlang des Bearbeitungspfads (t), der durch die Behandlungssteuerungsdaten definiert ist, unter Verwendung der ersten Tiefenscankomponente (z1) und der zweiten Tiefenscankomponente (z2) zu bestimmen.

8. Ophthalmologische Vorrichtung (1) nach einem der Ansprüche 6 oder 7, wobei die Schaltung (10) dazu konfiguriert ist, die Durchführbarkeit des Tiefenscans auszuführen, indem sie das Scannersystem (13) so steuert, dass es den Brennpunkt (S) unter Verwendung der ersten Tiefenscankomponente (z1) und der zweiten Tiefenscankomponente (z2) entlang des durch die Behandlungssteuerungsdaten definierten Bearbeitungspfads (t) bewegt, während die Laserquelle (11) in mindestens einen der folgenden Zustände versetzt wird: einen deaktivierten Zustand oder eine reduzierte Energie ohne jegliche Auswirkung auf das Augengewebe.

9. Ophthalmologische Vorrichtung (1) nach einem der Ansprüche 1 bis 8, wobei die ersten Scan-Leistungsmerkmale eine erste maximale Tiefenscangeschwindigkeit oder -frequenz umfassen; die zweiten Scan-Leistungsmerkmale eine zweite maximale Tiefenscangeschwindigkeit oder -frequenz umfassen, die schneller ist als die erste maximale Tiefenscangeschwindigkeit oder -frequenz; und die Schaltung (10) dazu konfiguriert ist, die erste Tiefenscankomponente (z1) unter Verwendung der ersten maximalen Tiefenscangeschwindigkeit oder -frequenz zu bestimmen, und um die zweite Tiefenscankomponente (z2) unter Verwendung der zweiten maximalen Tiefenscangeschwindigkeit oder -frequenz zu bestimmen.

10. Ophthalmologische Vorrichtung (1) nach einem der Ansprüche 1 bis 9, wobei die ersten Scan-Leistungsmerkmale eine erste maximale Amplitude der Tiefenmodulation bei einer bestimmten Geschwindigkeit oder Frequenz der Tiefenmodulation umfassen; die zweiten Scan-Leistungsmerkmale eine zweite maximale Amplitude der Tiefenmodulation bei der bestimmten Geschwindigkeit oder Frequenz der Tiefenmodulation umfassen, wobei die zweite maximale Amplitude der Tiefenmodulation kleiner ist als die erste maximale Amplitude der Tiefenmodulation in einem vergleichsweise niedrigeren dynamischen Leistungsbereich und die zweite maximale Amplitude der Tiefenmodulation größer ist als die erste maximale Amplitude der Tiefenmodulation in einem vergleichsweise höheren dynamischen Leistungsbereich; wobei die Schaltung (10) dazu konfiguriert ist, die erste Tiefenscankomponente (z1) unter Verwendung der ersten maximalen Amplitude der Tiefenmodulation zu bestimmen, und um die zweite Tiefenscankomponente (z2) unter Verwendung der zweiten maximalen Amplitude der Tiefenmodulation zu bestimmen.

11. Ophthalmologische Vorrichtung (1) nach einem der Ansprüche 1 bis 10, wobei die ersten Scan-Leistungsmerkmale mindestens eines der folgenden Merkmale umfassen: eine erste maximale Beschleunigung der Tiefenmodulation oder eine erste maximale Geschwindigkeit der Beschleunigung der Tiefenmodulation; und wobei die zweiten Scan-Leistungsmerkmale mindestens eines der folgenden Merkmale umfassen: eine zweite maximale Beschleunigung der Tiefenmodulation, die größer ist als die erste maximale Beschleunigung der Tiefenmodulation, oder eine zweite maximale Geschwindigkeit der Beschleunigung der Tiefenmodulation, die größer ist als die erste maximale Geschwindigkeit der Beschleunigung der Tiefenmodulation; und wobei die Schaltung (10) dazu konfiguriert ist, die erste Tiefenscankomponente (z1) unter Verwendung der ersten maximalen Beschleunigung oder der ersten maximalen Geschwindigkeit der Beschleunigung zu bestimmen, und um die zweite Tiefenscankomponente (z2) unter Verwendung der zweiten maximalen Beschleunigung oder der zweiten maximalen Geschwindigkeit der Beschleunigung zu bestimmen.

12. Ophthalmologische Vorrichtung (1) nach einem der Ansprüche 1 bis 11, wobei die ophthalmologische Vorrichtung (1) ferner eine Patientenkontaktfläche (18) umfasst, die eine zentrale Achse (m) aufweist und konfiguriert ist, um das fokussierende optische Modul (12) auf dem Auge (2) zu fixieren; und wobei die Schaltung (10) ferner dazu konfiguriert ist, im Falle einer Neigung des Auges (2) in Bezug auf die zentrale Achse (m) der Patientenkontaktfläche (18) die Behandlungssteuerungsdaten anzupassen, die gekrümmte Behandlungsoberfläche (C) entsprechend der Neigung des Auges (2) zu neigen, bevor die Tiefenscananforderung bestimmt wird, und die angepassten Behandlungssteuerungsdaten zu verwenden, um die Tiefenscananforderung zu bestimmen und die Tiefenscananforderung in die erste Tiefenscankomponente (z1) und die zweite Tiefenscankomponente (z2) zu unterteilen.

13. Ophthalmologische Vorrichtung (1) nach einem der Ansprüche 1 bis 12, wobei das Scannersystem (13) dazu konfiguriert ist, den Brennpunkt (S) entlang eines spiralförmigen Bearbeitungspfades (t) zu bewegen.

14. Ophthalmologische Vorrichtung (1) nach einem der Ansprüche 1 bis 13, wobei das x/y-Scannersystem (133) einen ersten x/y-Scanner (1331) umfasst, der dazu konfiguriert ist, den Brennpunkt (S) mit einer Vorschubgeschwindigkeit entlang einer Vorschublinie (w) des Bearbeitungspfads (t) zu bewegen, und wobei das x/y-Scannersystem (133) einen zweiten x/y-Scanner (1332) umfasst, der dazu konfiguriert ist, den Brennpunkt (S) mit einer Scangeschwindigkeit, die höher ist als die Vorschubgeschwindigkeit, entlang einer Scanlinie (r) zu bewegen, die quer zur Vorschublinie (w) des Bearbeitungspfads (t) verläuft.

15. Ophthalmologische Vorrichtung (1) nach einem der Ansprüche 1 bis 14, wobei der erste z-Scanner (131) einen ersten Aktuator (1310) umfasst, der zweite z-Scanner (132) einen zweiten Aktuator (1320) umfasst, und die Schaltung (10) dazu konfiguriert ist, eine Phasendifferenz zwischen der Betätigung durch den ersten Aktuator (1310) und der Betätigung durch den zweiten Aktuator (1320) zu bestimmen, und für die erste Tiefenscankomponente (z1) ein erstes Steuersignal für den ersten Aktuator (1310) und für die zweite Tiefenscankomponente (z2) ein zweites Steuersignal für den zweiten Akuator (1320) unter Verwendung der Phasendifferenz zu erzeugen.

16. Computerprogrammprodukt, umfassend ein nichtflüchtiges computerlesbares Medium, auf dem Computerprogrammcode zur Kontrolle eines Prozessors (100) einer ophthalmologischen Vorrichtung (1) gespeichert ist, die eine Laserquelle (11) umfasst, die zum Erzeugen eines gepulsten Laserstrahls (B) konfiguriert ist, ein fokussierendes optisches Modul (12), das eine Projektionsachse (z) aufweist und dazu konfiguriert ist, den gepulsten Laserstrahl (B) auf einen Brennpunkt (S) im Augengewebe zu fokussieren, und ein Scannersystem (13), das einen ersten z-Scanner (131) umfasst, der dazu konfiguriert ist, eine Tiefe (z) des Brennpunkts (S) entlang der Projektionsachse (p) mit ersten Scan-Leistungsmerkmalen zu modulieren, welche die dynamischen Tiefenscanfähigkeiten des ersten z-Scanners (131) angeben, einen zweiten z-Scanner (132), der dazu konfiguriert ist, die Tiefe (z) des Brennpunkts (S) entlang der Projektionsachse (p) mit zweiten Scan-Leistungsmerkmalen zu modulieren, welche die dynamischen Tiefenscanfähigkeiten des zweiten z-Scanners (132) anzeigen, die größer als die dynamischen Tiefenscanfähigkeiten des ersten z-Scanners (131) sind, und ein x/y-Scannersystem (133), das dazu konfiguriert ist, den Brennpunkt (S) in Richtungen senkrecht zur Projektionsachse (p) zu bewegen; wobei der Computerprogrammcode zur Kontrolle des Prozessors (100) konfiguriert ist, wie etwa, dass der Prozessor (100):
Behandlungssteuerungsdaten verwendet, um das Scannersystem (13) zu steuern, um den Brennpunkt (S) in dem Augengewebe zu Zielorten entlang eines Bearbeitungspfades (t) zu bewegen, der durch die Behandlungssteuerungsdaten definiert ist, um eine gekrümmte Behandlungsoberfläche (C) in dem Augengewebe zu bearbeiten;
**dadurch gekennzeichnet, dass** der Computerprogrammcode ferner zur Kontrolle des Prozessors (100) konfiguriert ist, wie etwa, dass der Prozessor (100):
aus den Behandlungssteuerungsdaten eine Tiefenscananforderung bestimmt, die eine Modulation der Tiefe (z) des Brennpunkts (S) entlang des durch die Behandlungssteuerungsdaten definierten Bearbeitungspfads (t) darstellt;
die Tiefenscananforderung in eine erste Tiefenscankomponente (z1) für den ersten z-Scanner (131) und eine zweite Tiefenscankomponente (z2) für den zweiten z-Scanner (132) teilt;
den ersten Z-Scanner (131) unter Verwendung der ersten Tiefenscankomponente (z1) steuert; und
den zweiten Z-Scanner (132) unter Verwendung der zweiten Tiefenscankomponente (z2) steuert.

## Revendications

1. Un dispositif ophtalmologique (1) pour le traitement d'une face de traitement incurvée (C) dans le tissu oculaire, le dispositif ophtalmologique (1) comprenant :
une source laser (11) configurée pour générer un faisceau laser pulsé (B) ;
un module optique de focalisation (12) ayant un axe de projection (z) et configuré pour faire converger le faisceau laser pulsé (B) vers un point focal (S) dans ou sur le tissu oculaire ;
un système de balayage (13) comprenant un premier scanner z (131) configuré pour moduler une profondeur (z) du point focal (S) le long de l'axe de projection (p) avec des caractéristiques de performance de premier balayage, indiquant des capacités de balayage dynamique en profondeur du premier scanner z (131), un second scanner z (132) configuré pour moduler la profondeur (z) du point focal (S) le long de l'axe de projection (p) avec des caractéristiques de performance de second balayage, indiquant des capacités de balayage dynamique en profondeur du second scanner z (132) qui sont supérieures aux capacités de balayage dynamique en profondeur du premier scanner z (131), et un système de balayage x/y (133) configuré pour déplacer le point focal (S) dans des directions normales à l'axe de projection (p) ; et
un circuit (10) configuré pour commander le système de balayage (13) afin de déplacer le point focal (S) vers des positions cibles sur la face de traitement incurvée (C) le long de la trajectoire de traitement (t) défini par des données de commande de traitement,
**caractérisé par le fait que** le circuit est en outre configuré pour
déterminer à partir des données de commande de traitement une exigence de balayage en profondeur, représentant la modulation de la profondeur (z) du point focal (S) le long de la trajectoire de traitement (t) définie par les données de commande de traitement,
diviser l'exigence de balayage en profondeur en une première composante de balayage en profondeur (z1) pour le premier scanner z (131) et une seconde composante de balayage en profondeur (z2) pour le second scanner z (132),
commander le premier scanner z (131) à l'aide de la première composante de balayage en profondeur (z1), et
commander le second scanner z (132) à l'aide de la seconde composante de balayage en profondeur (z2).

2. Le dispositif ophtalmologique (1) de la revendication 1, dans lequel le circuit (10) est configuré pour déterminer la première composante de balayage en profondeur (z1) et la seconde composante de balayage en profondeur (z2), en utilisant les caractéristiques de performance de premier balayage et les caractéristiques de performance de second balayage.

3. Le dispositif ophtalmologique (1) de la revendication 1, dans lequel le circuit (10) est configuré pour déterminer à partir des données de commande de traitement une composante sphérique de la face de traitement incurvée (C) et une composante complémentaire pour la face de traitement incurvée (C), la composante complémentaire complétant la composante sphérique pour constituer la face de traitement incurvée (C), et de diviser l'exigence de balayage en profondeur en la première composante de balayage en profondeur (z1), comme requis pour la modulation de la profondeur (z) du point focal (S) pour la composante sphérique, et la seconde composante de balayage en profondeur (z2), comme requis pour la modulation de la profondeur (z) du point focal (S) pour la composante complémentaire.

4. Le dispositif ophtalmologique (1) de la revendication 1, dans lequel la détermination de l'exigence de balayage en profondeur comprend la détermination de la dynamique requise de la modulation de la profondeur (z) du point focale (S) le long de la trajectoire de traitement (t) défini par les données de commande de traitement ; et le circuit (10) est configuré pour déterminer la première composante de balayage en profondeur (z1) en utilisant les caractéristiques de performance de premier balayage et la dynamique requise, et pour déterminer la deuxième composante de balayage en profondeur (z2) en utilisant les caractéristiques de performance de second balayage et la dynamique requise.

5. Le dispositif ophtalmologique (1) de la revendication 4, dans lequel la dynamique requise comprend au moins l'un des éléments suivants : une vitesse de balayage de profondeur requise, une fréquence de balayage de profondeur requise, une amplitude de modulation de profondeur requise à une vitesse particulière de la modulation de profondeur, une amplitude de modulation de profondeur requise à une fréquence particulière de la modulation de profondeur, une accélération de la modulation de profondeur requise, ou une vitesse de l'accélération de la modulation de profondeur requise.

6. Le dispositif ophtalmologique (1) de l'une des revendications 4 ou 5, dans lequel le circuit (10) est configuré pour déterminer la faisabilité du balayage en profondeur en vérifiant si l'exigence de balayage en profondeur est réalisable pour la dynamique requise, sans dépasser au moins l'une des capacités de balayage dynamique en profondeur du premier scanner z ( 131 ) ou des capacités de balayage dynamique en profondeur du second scanner z (132), et pour ajuster les données de commande de traitement afin de réduire ou de faire varier une vitesse de déplacement du point focal (S) le long de la trajectoire de traitement (t), au cas où la faisabilité du balayage en profondeur n'est pas affirmée.

7. Le dispositif ophtalmologique (1) de la revendication 6, dans lequel le circuit (10) est configuré pour déterminer la faisabilité du balayage en profondeur en calculant une simulation de déplacement du point focal (S) le long de la trajectoire de traitement (t), définie par les données de commande de traitement, à l'aide de la première composante de balayage en profondeur (z1) et de la seconde composante de balayage en profondeur (z2).

8. Le dispositif ophtalmologique (1) de l'une des revendications 6 ou 7, dans lequel le circuit (10) est configuré pour effectuer la faisabilité du balayage en profondeur en commandant le système de balayage (13) pour déplacer le point focal (S) le long de la trajectoire de traitement (t), définie par les données de commande de traitement, en utilisant la première composante de balayage en profondeur (z1) et la seconde composante de balayage en profondeur (z2), tout en réglant la source laser (11) sur au moins l'un des éléments suivants : un état désactivé ou une énergie réduite sans aucun effet sur le tissu oculaire.

9. Le dispositif ophtalmologique (1) de l'une des revendications 1 à 8, dans lequel les caractéristiques de performance de premier balayage comprennent une première vitesse ou fréquence maximale de balayage en profondeur ; les caractéristiques de performance de second balayage comprennent une seconde vitesse ou fréquence maximale de balayage en profondeur qui est plus rapide que la première vitesse ou fréquence maximale de balayage en profondeur ; et le circuit (10) est configuré pour déterminer la première composante de balayage en profondeur (z1) en utilisant la première vitesse ou fréquence maximale de balayage en profondeur, et pour déterminer la seconde composante de balayage en profondeur (z2) en utilisant la seconde vitesse ou fréquence maximale de balayage en profondeur.

10. Le dispositif ophtalmologique (1) de l'une des revendications 1 à 9, dans lequel les caractéristiques de performance de premier balayage comprennent une première amplitude maximale de modulation de profondeur à une vitesse ou une fréquence particulière de la modulation de profondeur ; les caractéristiques de performance de second balayage comprennent une seconde amplitude maximale de modulation de profondeur à la vitesse ou à la fréquence particulière de la modulation de profondeur, la seconde amplitude maximale de modulation de profondeur étant plus petite que la première amplitude maximale de modulation de profondeur dans une plage de performance dynamique comparativement inférieure, et la seconde amplitude maximale de modulation de profondeur étant plus grande que la première amplitude maximale de modulation de profondeur dans une plage de performance dynamique comparativement supérieure ; et le circuit (10) est configuré pour déterminer la première composante de balayage en profondeur (z1) à l'aide de la première amplitude maximale de modulation de profondeur, et pour déterminer la seconde composante de balayage en profondeur (z2) à l'aide de la seconde amplitude maximale de modulation de profondeur.

11. Le dispositif ophtalmologique (1) de l'une des revendications 1 à 10, dans lequel les caractéristiques de performance de premier balayage comprennent au moins l'un des éléments suivants : une première accélération maximale de la modulation de profondeur, ou une première vitesse maximale de l'accélération de la modulation de profondeur ; les caractéristiques de performance de second balayage comprennent au moins l'un des éléments suivants : une deuxième accélération maximale de la modulation de profondeur, étant plus grande que la première accélération maximale de la modulation de profondeur, ou une deuxième vitesse maximale de l'accélération de la modulation de profondeur, étant plus grande que la première vitesse maximale de l'accélération de la modulation de profondeur ; et le circuit (10) est configuré pour déterminer la première composante de balayage en profondeur (z1) à l'aide de la première accélération maximale ou de la première vitesse maximale de l'accélération, et pour déterminer la deuxième composante de balayage en profondeur (z2) à l'aide de la deuxième accélération maximale ou de la deuxième vitesse maximale de l'accélération.

12. Le dispositif ophtalmologique (1) de l'une des revendications 1 à 11, dans lequel le dispositif ophtalmologique (1) comprend en outre une interface patient (18) présentant un axe central (m) et configurée pour fixer le module optique de focalisation (12) sur l'œil (2) ; et le circuit (10) est en outre configuré, en cas d'inclinaison de l'œil (2) par rapport à l'axe central (m) de l'interface patient (18), pour adapter les données de commande de traitement afin d'incliner la surface de traitement incurvée (C) correspondant à l'inclinaison de l'œil (2), avant de déterminer l'exigence de balayage en profondeur, et pour utiliser les données de commande de traitement adaptées afin de déterminer l'exigence de balayage en profondeur et de diviser l'exigence de balayage en profondeur en la première composante de balayage en profondeur (z1) et la seconde composante de balayage en profondeur (z2).

13. Le dispositif ophtalmologique (1) de l'une des revendications 1 à 12, dans lequel le système de balayage (13) est configuré pour déplacer le point focal (S) le long d'une trajectoire de traitement (t) en forme de spirale.

14. Le dispositif ophtalmologique (1) de l'une des revendications 1 à 13, dans lequel le système de balayage x/y (133) comprend un premier scanner x/y (1331), configuré pour déplacer le point focal (S) avec une vitesse d'avance le long d'une ligne d'avance (w) de la trajectoire de traitement (t), et le système de balayage x/y (133) comprend un second scanner x/y (1332), configuré pour déplacer le point focal (S) avec une vitesse de balayage, qui est plus grande que la vitesse d'avance, le long d'une ligne de balayage (r) s'étendant transversalement par rapport à la ligne d'avance (w) de la trajectoire de traitement (t).

15. Le dispositif ophtalmologique (1) de l'une des revendications 1 à 14, dans lequel le premier scanner z (131) comprend un premier actionneur (1310), le second scanner z (132) comprend un second actionneur (1320), et le circuit (10) est configuré pour déterminer une différence de phase entre l'actionnement par le premier actionneur (1310) et l'actionnement par le second actionneur (1320), et pour générer, pour la première composante de balayage en profondeur (z1), un premier signal de commande pour le premier actionneur (1310) et, pour la seconde composante de balayage en profondeur (z2), un second signal de commande pour le second actionneur (1320), en utilisant la différence de phase.

16. Produit programme d'ordinateur comprenant un support lisible par ordinateur non transitoire sur lequel est stocké un code programme d'ordinateur pour commander un processeur (100) d'un dispositif ophtalmologique (1) qui comprend une source laser (11) configurée pour générer un faisceau laser pulsé (B), un module optique de focalisation (12) ayant un axe de projection (z) et étant configuré pour faire converger le faisceau laser pulsé (B) vers un point focal (S) dans le tissu oculaire, et un système de balayage (13) comprenant un premier scanner z (131), configuré pour moduler une profondeur (z) du point focal (S) le long de l'axe de projection (p) avec des caractéristiques de performance de premier balayage, indiquant les capacités de balayage dynamique en profondeur du premier scanner z (131), un second scanner z (132), configuré pour moduler la profondeur (z) du point focal (S) le long de l'axe de projection (p) avec des caractéristiques de performance de second balayage, indiquant les capacités de balayage dynamique en profondeur du second scanner z (132) qui sont supérieures aux capacités de balayage dynamique en profondeur du premier scanner z (131), et un système de balayage x/y (133) configuré pour déplacer le point focal (S) dans des directions normales à l'axe de projection (p) ; par lequel le code programme d'ordinateur est configuré pour commander le processeur (100) de sorte que le processeur (100) :
utilise les données de commande de traitement pour commander le système de balayage (13) afin de déplacer le point focal (S) dans le tissu oculaire vers des positions cibles le long d'une trajectoire de traitement (t), défini par les données de commande de traitement pour traiter une face de traitement incurvée (C) dans le tissu oculaire ;
**caractérisé par le fait que** le code programme d'ordinateur est en outre configuré pour commander le processeur (100) de sort que le processeur (100) :
détermine à partir des données de commande de traitement une exigence de balayage en profondeur, représentant la modulation de la profondeur (z) du point focal (S) le long de la trajectoire de traitement (t) défini par les données de commande de traitement ;
divise l'exigence de balayage en profondeur en une première composante de balayage en profondeur (z1) pour le premier scanner z (131) et une seconde composante de balayage en profondeur (z2) pour le second scanner z (132) ;
commande le premier scanner z (131) à l'aide de la première composante de balayage en profondeur (z1) ; et
commande le second scanner z (132) à l'aide de la seconde composante de balayage en profondeur (z2).
